# EUROPEAN PATENT APPLICATION

(11) **EP 3 970 866 A1**
(43) Date of publication of application: **23.03.2022**
(21) Application number: 20805910.5
(22) Date of filing: 15.05.2020
(51) Int. Cl.: B06B 1/04, B62D 1/06, G01C 21/36, G06F 3/01

(54) **FORCE SENSATION MODULE AND HAPTIC DEVICE**

(30) Priority: 15.05.2019 JP 2019092135
(71) Applicant: NTT Communications Corporation, Tokyo 100-8019 (JP)
(72) Inventor: TSUDA, Yuki, Tokyo 100-8019 (JP)
(74) Representative: Nederlandsch Octrooibureau
(86) International application number: PCT/JP2020/019419
(87) International publication number: WO 2020/230887

(57) **Abstract**

A force module and a haptic device configured to prevent the vibration property from being diminished are offered.

The force module according to one aspect includes a force sensation creating device configured to create a kinesthetic illusion through asymmetric vibration; and a supporting portion arranged between the force sensation creating device and a target portion to which the force sensation creating device is attached, and configured to support the force sensation creating device with respect to the target portion in such a manner as to allow for vibration.

## Description

The present invention generally relates to a force module and a haptic device configured to create an experience of a kinesthetic illusion of being pulled.

### BACKGROUND

A force sensation creating device has been known which causes a user to perceive a sensation of being pulled by linearly reciprocating a weight and producing asymmetric vibrations. The force sensation creating device may produce asymmetric vibrations through eccentric translational movement of the weight so that the user will perceive a sensation of being pulled in a specific direction. The force sensation creating device may be formed, for example, into a gripping type that the user can grip in one hand or a lighter type that the user can pinch with his/her finger tips.

### CITATION LIST

### [PATENT LITERATURE]

[Patent Literature 1] Japanese Patent No. 6379075

### SUMMARY

### TECHNICAL PROBLEM

Difficulty arises in producing a targeted kinesthetic sensation when a force sensation creating device is attached and fixed with an adhesion tape or the like directly to a target portion of an apparatus of any type where it is touched by a hand or fingers, which tends to weaken or cancel the vibrations, diminishing the vibration property.

The purpose of the present invention is to provide a force module and a haptic device which can prevent the vibration property from being diminished.

### SOLUTION TO PROBLEM

The force module according to the first aspect of the present invention includes a force sensation creating device configured to create a kinesthetic illusion through asymmetric vibrations; and a supporting portion arranged between the force sensation creating device and a target portion to which the force sensation creating device is attached, the supporting portion being configured to support the force sensation creating device with respect to the target portion in such a manner as to allow for vibration. According to this aspect, the force sensation creating device is prevented from losing its vibration property.

According to another aspect, the supporting portion is a base configured to be deformable.

According to this aspect, with the deformable base, the force sensation creating device can be supported with a simple configuration in a manner that allows for vibration.

According to another aspect, the force sensation creating device is configured to generate asymmetric vibration in such a manner as to provide a being-pulled sensation in a predetermined guiding direction, the target portion is an operation member manually manipulated by a user, and the guiding direction of the force sensation creating device is established along a manipulation direction of the target portion.

According to this aspect, the user can be informed of the direction of the user's manual manipulation through a force sensation.

According to another aspect, the target portion is a steering wheel that is rotationally manipulated, and the force sensation creating device is arranged on an upper side of the steering wheel with respect to a rotational shaft of the steering wheel.

According to this aspect, by providing a powerful kinesthetic illusion, the rotational manipulation of the steering wheel can be assisted.

According to another aspect, the supporting portion includes an elastically deformable portion configured in an elastically deformable manner.

According to this aspect, with the supporting portion being elastically deformed, the vibration property can be maintained.

According to another aspect, the supporting portion has a hollow portion.

According to this aspect, the module can be configured, as a whole, to have a simple configuration and to be lightweight.

According to another aspect, the force module includes a cover member having flexibility and configured to cover the force sensation creating device and the supporting portion together with the target portion of attachment.

According to this aspect, the force sensation creating device can be supported with a simple configuration in such a manner that the vibration can be conveyed.

According to another aspect, the force sensation creating device comprises a vibrator, a linking mechanism connected to the vibrator, a sliding mechanism configured to guide a moving direction of the linking mechanism and the vibrator, a rotator connected to the linking mechanism, and a controller configured to drive the rotator to rotate.

According to this aspect, an asymmetric vibration can be generated through the rotation of the rotator.

According to another aspect, the supporting portion is formed of urethane foam.

According to this aspect, the vibration property can be maintained with a simple and lightweight member.

According to another aspect, the supporting portion is formed of a flexible band member and shaped into a ring having a hollow portion.

According to this aspect, the vibration property can be maintained with a simple and lightweight member.

According to another aspect, the supporting portion is sandwiched by, or adhered with an adhesive to, the force sensation creating device and the target portion.

According to this aspect, the being-pulled sensation creating device can be held by the target portion with a simple configuration, without losing the vibration property.

According to another aspect, the target portion is a steering wheel of a vehicle equipped with a car navigation system, and the force module comprises a controller configured to drive the force sensation creating device in such a manner as to create a being-pulled sensation based on a route indicated by the car navigation system.

According to this aspect, the invention can lead the driver by prompting the driver to perform a manipulation based on the route provided by the car navigation system.

According to another aspect, the target portion is a steering wheel of a vehicle, and the force module is provided in an area of the steering wheel where a thumb, an index finger, or a middle finger of the user is placed.

According to this aspect, with the force module arranged at a position where the thumb, index finger, or middle finger can touch, the being-pulled effect in the direction of turning the steering wheel can be created.

The haptic device according to another aspect includes a target portion, and a force module including a force sensation creating device arranged in the target portion and configured to create a kinesthetic illusion through asymmetric vibration and a supporting portion arranged between the target portion and the force sensation creating device and configured to support the force sensation creating device with respect to the target portion in such a manner as to allow for vibration.

According to this aspect, the force module can be easily mounted onto various types of target portions.

According to another aspect, the target portion is an operation member that is manually manipulated, and the force module is arranged such that a vibration direction thereof is established along a manipulation direction.

According to this aspect, the force module can assist the manual manipulation of the operation member.

### ADVANTAGEOUS EFFECTS OF INVENTION

The present invention can offer a force module and a haptic device that can maintain a desired kinesthetic sensation.

### BRIEF DESCRIPTION OF THE DRAWINGS

FIG. 1 is a diagram for explaining the configuration of a steering wheel including a being-pulled sensation module according to the first embodiment.
FIG. 2 is a diagram for explaining the being-pulled sensation module.
FIG. 3 is a diagram for explaining the being-pulled sensation module in use.
FIG. 4 is a diagram for explaining the being-pulled sensation module in use.
FIG. 5 is a diagram for explaining the operation of the being-pulled sensation module.
FIG. 6 is a diagram for explaining the configuration of a steering wheel including a being-pulled sensation module according to another embodiment.
FIG. 7 is a diagram for explaining the configuration of a haptic device including a being-pulled sensation module according to another embodiment.
FIG. 8 is a diagram for explaining a being-pulled sensation module according to another embodiment.
FIG. 9 is a diagram for explaining a being-pulled sensation module according to another embodiment.

### DETAILED DESCRIPTION

The embodiments will be explained with reference to the drawings. Components that are the same as or similar to already explained components are provided with the same or similar reference signs, and overlapping explanations will be practically omitted.

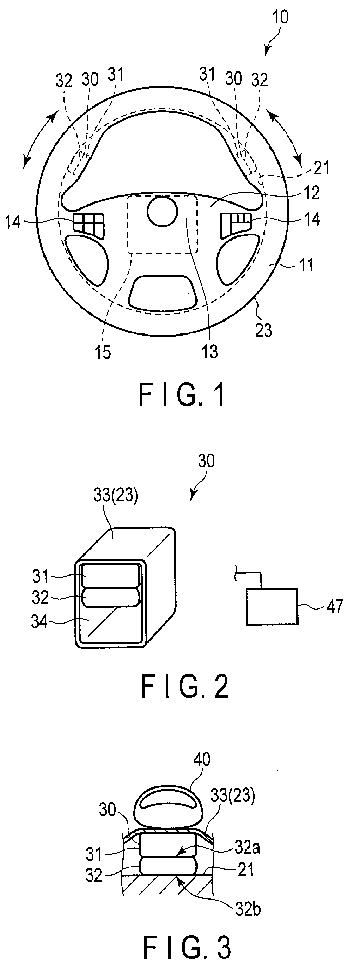

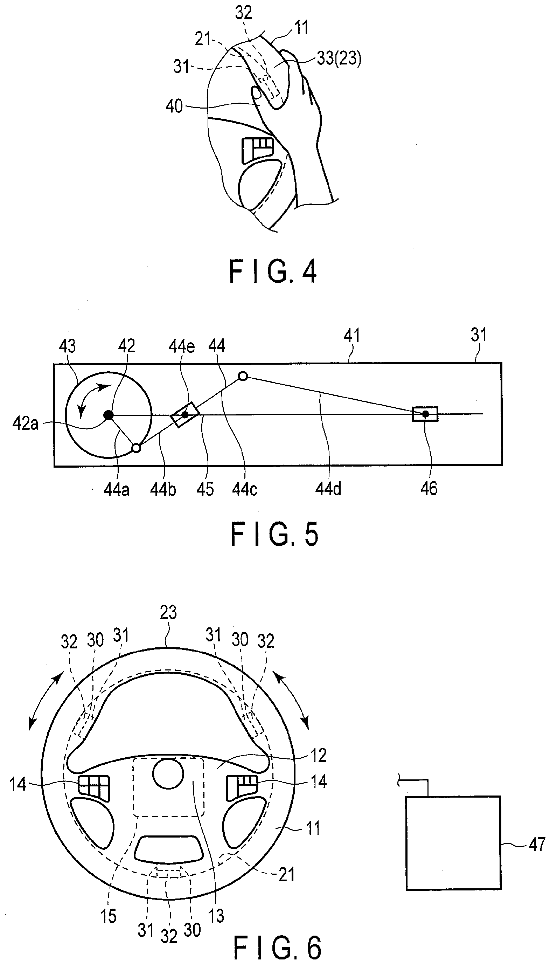

FIG. 1 is a drawing for explaining a steering wheel 10, which serves as a haptic device according to the first embodiment. FIG. 2 is a drawing for explaining a being-pulled sensation module 30, which is one example of a force module. FIGS. 3 and 4 are drawings for explaining the being-pulled sensation module 30 and steering wheel 10 in use. FIG. 5 is a drawing for explaining the operation of a being-pulled sensation creating device 31, which serves as a force sensation creating device.

In the present embodiment, an example is described in which the being-pulled sensation module 30, which is a force module, is arranged in the steering wheel 10, which is a haptic member that is mounted on a vehicle equipped with a car navigation system. The haptic device is a device that the user touches with a hand or fingers 40 or an operation member that the user manipulates. For instance, the device may be a member grasped with the hand or all the fingers 40, a member pinched with finger tips, or a member touched by part of the hand or finger 40.

The steering wheel 10 includes a circular rim 11, and a panel portion 12 arranged at the center side of the rim 11. An opening is formed between the rim 11 and panel portion 12, through which the user places the hand or fingers 40. The steering wheel 10 is connected to the driving system of the vehicle by way of a steering shaft so that the user can operate the driving system and steer the vehicle by holding the steering wheel 10 to perform a rotational manipulation.

The panel portion 12 includes a base panel 13 in which a circuit board 15 and wiring are contained, and interface panels 14 arranged on the two sides of the base panel 13 and having different switches. For instance, input switches for manipulating the driving system and audio system, and a display unit for displaying LED lights and various kinds of data are provided on the interface panels 14.

The rim 11 includes a rim frame 21, which is an attachment target, being-pulled sensation modules 30 arranged at predetermined positions of the rim frame 21, and a rim cover 23 that covers the rim frame 21 and being-pulled sensation modules 30. The rim frame 21 may be formed of a metal, such as steel, and shaped into a ring. The being-pulled sensation modules 30 are arranged on the surface of the rim frame 21 at predetermined positions on one side portion and the other side portion of the rim frame 21 with respect to the rotational shaft of the steering wheel 10, in the area of the rim frame 21 above the rotational shaft.

A being-pulled sensation module 30 includes a being-pulled sensation creating device 31, a base 32 that serves as a supporting portion, and a cover member 33. The being-pulled sensation module 30 includes a space 34 between the cover member 33 and base 32, in which the attachment target portion is arranged. In the present embodiment, an exemplary configuration in which the rim frame 21 is arranged in the space 34, and the cover member 33 is formed as part of the rim cover 23, is illustrated. Each of the being-pulled sensation modules 30 may be arranged on the inner peripheral side of the rim frame 21 that is the target portion. A being-pulled sensation module 30 may be positioned where the user's thumb, index finger, or middle finger is placed on the rim frame 21. The user holding the rim 11 places his/her thumbs, index fingers, or middle fingers on the surfaces of the being-pulled sensation modules 30.

As illustrated in FIG. 5, the being-pulled sensation creating device 31 may include a casing 41, a driving unit 42, a rotator 43, a linking mechanism 44, a sliding mechanism 45, a vibrator 46, and a controller 47 connected to the driving unit 42.

The casing 41 may be formed of a synthetic resin into a rectangular box. The casing 41 is arranged in a depression for storage, which is formed on the inner side of the rim frame 21.

The driving unit 42 may be a rotary motor, having a rotary shaft 42a. The driving unit 42 is connected to the controller 47 to rotate the rotator 43 under the control of the controller 47.

The rotator 43 may be formed into a disk, and driven to rotate by the driving unit 42. The linking mechanism 44 is connected to the rotator 43 at a specific position away from the rotary shaft 42a.

The linking mechanism 44 is configured by mutually coupling a plurality of beam members 44a to 44d in a rotatable or slidable manner. One end of the linking mechanism 44 is connected to the rotator 43, and the other end of the linking mechanism 44 is connected to the vibrator 46.

The sliding mechanism 45 includes a slide rail engaged with the vibrator 46 and a slide point 44e set at a specific position of the linking mechanism 44, and regulates the operation of the slide point 44e of the linking mechanism 44 and the vibrator 46 in a manner such that the moving direction of the slide point 44e and the vibrator 46 matches the predetermined sliding direction.

The vibrator 46 is a weight having a specific mass, and is supported by the linking mechanism 44 and sliding mechanism 45 in such a manner as to reciprocate in a specific direction.

The controller 47 may be provided in the circuit board 15 . The controller 47, which includes a processor, operates the being-pulled sensation creating device 31 under specific operational conditions based on a predetermined program or a detected condition. The controller 47 drives the being-pulled sensation creating device 31 such that a being-pulled sensation can be created at a timing corresponding to the optimal route found by a car navigation system mounted on a vehicle and in a guiding direction also corresponding thereto. Specifically, the rotator 43 is driven to rotate in a manner such as to generate vibrations with its velocity in the guiding direction higher than the velocity in the opposite direction, and the user is prompted to manipulate the steering wheel accordingly. The controller 47 further drives the display unit to turn the light on, for example when the being-pulled sensation creating device 31 is operating, so that the user can be informed that the being-pulled sensation creating device 31 is in operation.

In the being-pulled sensation module 30, in accordance with the rotation of the rotator 43, the vibrator 46 performs a cyclic translational movement in a specific direction, demonstrating an uneven acceleration with the linking mechanism 44 and sliding mechanism 45. In this translational movement, the vibrator 46 moves, in accordance with the rotational direction of the rotator 43, at a high acceleration for a shorter time in a guiding direction in which a kinesthetic illusion is intended to be created, and at a low acceleration for a longer time in the opposite direction. In this manner, the user touching the system including the vibrator 46 perceives the kinesthetic illusion in the guiding direction.

The being-pulled sensation creating device 31 is arranged in an orientation such that the user can feel the sensation of being pulled in the manipulation direction, or rotational direction, of the steering wheel 10. In particular, the being-pulled sensation creating device 31 is arranged such that the sliding direction being the vibrating direction of the vibrator 46, which is the guiding direction in which a kinesthetic illusion is intended to be demonstrated, can be established along the tangential direction at the position of the target portion of the rim frame 21 where the being-pulled sensation creating device 31 is attached.

The base 32 is formed of a material having a larger softness and higher elasticity than that of the surface of the rim frame 21 that is the target portion, and configured in a deformable manner. For instance, the base 32 may be a hollow or solid spring member formed of urethane foam. The base 32 may have a first surface 32a and a second surface 32b, which face each other and have a specific thickness. The first surface 32a is formed to have the same shape as that of the bottom surface of the being-pulled sensation creating device 31, and is at least in part in contact with the being-pulled sensation creating device 31. The second surface 32b is at least in part in contact with the target portion. The base 32 may be adhered to the being-pulled sensation creating device 31 and rim frame 21 with an adhesive, or sandwiched without being adhered.

In the base 32, which is configured to be deformable, the first surface 32a and second surface 32b can be moved relative to each other. The base 32 deposited between the rigid rim frame 21 and the being-pulled sensation creating device 31 has a floating configuration for supporting the being-pulled sensation creating device 31. In this configuration, because the rim frame 21 and being-pulled sensation creating device 31 are separated from each other, the vibration of the being-pulled sensation creating device 31 can be conveyed without being cancelled.

The cover member 33 is formed of a soft material having flexibility such as fabric, synthetic fabric, and leather, and is fixed in such a manner as to convey vibrations. The cover member 33 is deformable so as to fit the outer shape of the being-pulled sensation creating device 31 and is configured to convey the vibrations to the hand or fingers 40.

In the above being-pulled sensation modules 30, the controller 47 rotates the rotator 43. When the rotator 43 rotates in the casing 41 of the being-pulled sensation creating device 31, the end of the linking mechanism 44 coupled with the rotator 43 rotates. In accordance with this, the vibrator 46 performs an eccentric reciprocation movement with the sliding mechanism 45 and linking mechanism 44. Here, the vibrator 46 exhibits an asymmetric reciprocation with different accelerations, rapidly in one orientation of the sliding direction and slowly in the other orientation. The difference in the acceleration in moving directions of the vibrator 46 creates, for the user touching the being-pulled sensation creating device 31, a sensation of being pulled in the sliding direction toward the orientation with a large acceleration.

In the being-pulled sensation module 30, the being-pulled sensation creating device 31 is supported on the attachment target by way of a deformable base 32. This prevents the vibrations of the being-pulled sensation creating device 31 from being attenuated or interfered with, thereby maintaining a desired being-pulled sensation. If the being-pulled sensation creating device 31 is fixed by direct adhesion or the like to an attachment target formed of a rigid material, the vibrations of the being-pulled sensation creating device 31 tend to be impaired by the attachment target. In addition, in the case of the steering wheel 10 vibrated by the traveling vehicle, the vibration property of the being-pulled sensation creating device 31 may be impaired, for example by the vibrations of the steering wheel 10 conveyed to the being-pulled sensation creating device 31. In contrast, with the support by a base 32 interposed according to the present embodiment, the vibrations are conveyed to the hand or fingers 40 without the vibration property being impaired, and a desired sensation can be provided.

The being-pulled sensation module 30 is applicable to various devices and appliances. When it is arranged on an operation member touched and manipulated with the hand or fingers, the direction and timing of the manipulation can be informed, thereby assisting the manipulation. For instance, according to the present embodiment, the being-pulled sensation module 30 may be attached to the steering wheel of a vehicle to create a being-pulled sensation based on the optimal route presented by a car navigation system or the like under the controller, thereby guiding the driver to a destination.

Furthermore, the position of the being-pulled sensation module 30 is determined to be in the upper area of the steering wheel 10, which can enhance the being-pulled sensation. In addition, the being-pulled sensation creating device 31 of the being-pulled sensation module 30, which is positioned where the thumb, index finger or middle finger can touch, can produce the being-pulled effect toward the direction of turning the steering wheel 10.

The above embodiment is illustrated simply as a concrete example for facilitating the understanding of the concept of the present invention, and is not intended to limit the scope of the invention. Addition, deletion, or replacement of structural components may be made without departing from the scope of the invention.

The attachment positions and number of being-pulled sensation modules 30 are not limited to the above embodiment. For instance, as illustrated in FIG. 6, a being-pulled sensation module 30 may be additionally arranged in the lower area of the steering wheel 10, or a plurality of being-pulled sensation modules 30 may be arranged at each position. Alternatively, a plurality of being-pulled sensation creating devices 31 may be provided in one being-pulled sensation module 30, or a plurality of guiding directions may be determined to have different vibration directions.

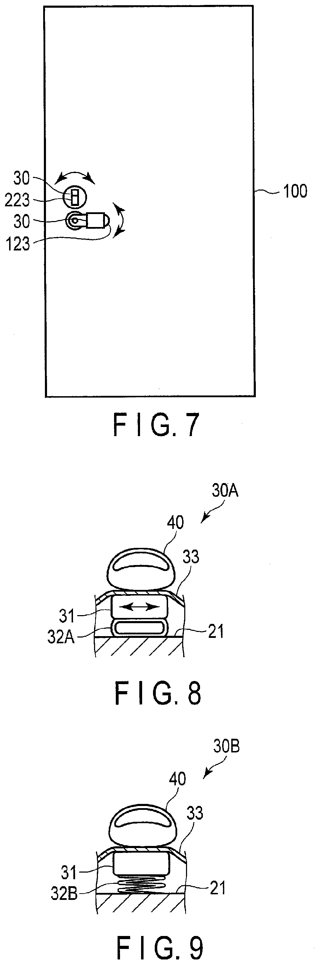

In the above embodiment, the steering wheel 10 has been described as an attachment-targeted haptic device, which is not a limitation. An attachment target may be any other devices and equipment. For instance, as illustrated in FIG. 7, the attachment may be made on a operation member rotated with the hand or fingers such as a knob 123 of a door 100 or a lock lever 223 of the door 100. For instance, the attachment may be made by inserting the knob 123 or lock lever 223 into the space 34 provided in the being-pulled sensation module 30. In this manner, a retrofit attachment to an existing device can be achieved. The guiding direction may be determined to be the rotational direction, which is the manipulation direction of the knob 123 or lever 223. In this manner, the opening/closing operation of the door 100 or a lock can be guided and assisted. In addition, a being-pulled sensation creating device 31 may be attached to various operation members such as those of console game machines by way of a base 32 to provide the devices with a guiding function for indicating the timing and direction of an operation.

In the above embodiment, an example of the being-pulled sensation creating device 31 arranged on the surface of the rim frame 21 and covered by the rim cover 23 has been introduced, which is not a limitation. For instance, the being-pulled sensation creating device 31 may be attached to the surface of the steering wheel 10 by way of the base 32 with the cover omitted so as to be externally exposed. Alternatively, the configuration may be such that a depression is formed in the rim frame 21 to store the being-pulled sensation creating device 31.

In the above embodiment, an exemplary configuration in which the target portion, base 32, and being-pulled sensation creating device 31 are covered by the cover member 33 so that the being-pulled sensation creating device 31 can be attached to the target portion by way of the base 32 has been introduced, which is not a limitation. The configuration may be such that the being-pulled sensation creating device 31 is fastened by a different supporting structure in such a manner as to allow for vibration. The being-pulled sensation creating device 31 may be partially or entirely exposed to the outside.

The configuration of the base is not limited to the exemplary configuration according to the embodiment. In addition to an elastically deformable configuration, various configurations that allow for conveyance of vibrations of the being-pulled sensation creating device 31 to the hand or finger 40, for example with a spring property, elasticity, toughness, flexibility, or shock absorption, can be adopted. According to another embodiment, as illustrated in FIG. 8, a being-pulled sensation module 30A includes a base 32A configured by forming a flexible band member into a hollow circle as an elastically deformable portion. According to still another embodiment, a being-pulled sensation module 30B in FIG. 9 includes a base 32B formed of a spring member as an elastically deformable portion. According to these embodiments, the being-pulled sensation creating device 31 of the being-pulled sensation modules 30A and 30B can be supported without impairing the vibration property. Thus, the being-pulled sensation modules 30A and 30B can realize perception of a desired being-pulled sensation in a manner similar to the being-pulled sensation module 30 according to the first embodiment. The base may be configured to amplify the vibrations.

The driving mechanism and power source of the being-pulled sensation creating device 31 are not limited to the above. For instance, a reciprocating motor or shaft motor may be adopted. In place of the linking mechanism or sliding mechanism, other mechanisms may be adopted.

In the above embodiment, an example in which the controller 47 is provided in the circuit board 15 inside the panel portion 12 of the steering wheel 10 has been presented, which is not a limitation. For instance, the controller 47 may be provided in an external device such as a smart phone or a vehicle-mounted ECU so as to be operated by the external device. The controller may be arranged in the vehicle-mounted ECU to control the being-pulled sensation creating device 31 in association with the control of the driving system.

The present invention should not be limited to the above-described embodiments, but may be modified without departing from the scope of the invention at the implementation stage. Each embodiment may be implemented in a suitable combination with others, and in such a case, combined effects can be attained. The above embodiments contain various inventions, and various inventions can be extracted from combinations of selected ones of the disclosed structural components. For instance, if the issue of the invention can be solved and effects can be attained even with some of the structural components of the embodiments omitted, the configuration without these components may be extracted as an invention.

### REFERENCE SIGNS LIST

- 10: Steering wheel (haptic device)
- 11: Rim
- 12: Panel portion
- 13: Base panel
- 14: Interface panel
- 15: Circuit board
- 21: Rim frame
- 23: Rim cover
- 30: Being-pulled sensation module (force module)
- 31: Being-pulled sensation creating device (force sensation creating device)
- 32: Base (supporting portion)
- 32a: First surface
- 32b: Second surface
- 33: Cover member
- 34: Space
- 40: Hand or Finger
- 41: Casing
- 42: Driving unit
- 42a: Rotary shaft
- 43: Rotator
- 44: Linking mechanism
- 44a to 44d: Beam members
- 44e: Slide point
- 45: Sliding mechanism
- 46: Vibrator
- 47: Controller
- 100: Door
- 123: Knob
- 223: Lever

## Claims

1. A force module comprising:
a force sensation creating device configured to create a kinesthetic illusion through asymmetric vibration; and
a supporting portion arranged between the force sensation creating device and a target portion to which the force sensation creating device is attached, the supporting portion being configured to support the force sensation creating device with respect to the target portion in such a manner as to allow for vibration.

2. The force module according to claim 1, wherein
the supporting portion is a base formed in a deformable manner.

3. The force module according to claim 1 or 2, wherein
the force sensation creating device is configured to generate asymmetric vibration in such a manner as to provide a being-pulled sensation in a predetermined guiding direction,
the target portion is an operation member manually manipulated by a user, and
the guiding direction of the force sensation creating device is established along a manipulation direction of the target portion.

4. The force module according to any one of claims 1 to 3, wherein
the target portion is a steering wheel that is rotationally manipulated, and
the force sensation creating device is arranged on an upper side of the steering wheel with respect to a rotational shaft of the steering wheel.

5. The force module according to any one of claims 1 to 4, wherein
the supporting portion includes an elastically deformable portion formed in an elastically deformable manner.

6. The force module according to any one of claims 1 to 5, wherein
the supporting portion has a hollow portion.

7. The force module according to any one of claims 1 to 6, comprising:
a cover member having flexibility and configured to cover the force sensation creating device and the supporting portion together with the target portion.

8. The force module according to any one of claims 1 to 7, wherein
the force sensation creating device comprises a vibrator, a linking mechanism connected to the vibrator, a sliding mechanism configured to guide a moving direction of the linking mechanism and the vibrator, a rotator connected to the linking mechanism, and a controller configured to drive the rotator to rotate.

9. The force module according to any one of claims 1 to 8, wherein
the supporting portion is formed of urethane foam.

10. The force module according to any one of claims 1 to 8, wherein
the supporting portion is formed of a flexible band member and shaped into a ring having a hollow portion.

11. The force module according to any one of claims 1 to 10, wherein
the supporting portion is sandwiched by, or adhered with an adhesive to, the force sensation creating device and the target portion.

12. The force module according to any one of claims 1 to 11, wherein
the target portion is a steering wheel of a vehicle equipped with a car navigation system, and
the force module comprises a controller configured to drive the force sensation creating device in such a manner as to create a being-pulled sensation based on a route indicated by the car navigation system.

13. The force module according to any one of claims 1 to 12, wherein
the target portion is a steering wheel of a vehicle, and
the force module is provided in an area of the steering wheel where a thumb, an index finger, or a middle finger of the user is placed.

14. A haptic device comprising:
a target portion; and
a force module including a force sensation creating device arranged in the target portion and configured to create a kinesthetic illusion through asymmetric vibration, and a supporting portion arranged between the target portion and the force sensation creating device and configured to support the force sensation creating device with respect to the target portion in such a manner as to allow for vibration.

15. The haptic device according to claim 14, wherein the target portion is provided in the operation member that is manually manipulated, and the force module is arranged such that a vibration direction thereof is established along the manipulation direction.

## Amended claims

### Amended claims under Art. 19.1 PCT

1. (Presently amended) A force module comprising:
a force sensation creating device configured to create a kinesthetic illusion through asymmetric vibration; and
a supporting portion arranged between the force sensation creating device and a target portion to which the force sensation creating device is attached, the supporting portion being configured to support the force sensation creating device with respect to the target portion in such a manner as to allow for vibration,
wherein the force sensation creating device is configured to generate asymmetric vibration in such a manner as to provide a being-pulled sensation in a predetermined guiding direction,
the target portion is an operation member manually manipulated by a user,
the guiding direction of the force sensation creating device is established along a manipulation direction of the target portion,
the target portion is a steering wheel that is rotationally manipulated, and
the force sensation creating device is arranged on an upper side of the steering wheel with respect to a rotational shaft of the steering wheel.

2. The force module according to claim 1,
wherein the supporting portion is a base formed in a deformable manner.

3. (Canceled)

4. (Canceled)

5. (Presently amended) The force module according to claim 1 or 2, wherein
the supporting portion includes an elastically deformable portion formed in an elastically deformable manner.

6. (Presently amended) The force module according to claim 1, 2 or 5, wherein
the supporting portion has a hollow portion.

7. (Presently amended) The force module according to claim 1, 2, 5 or 6, comprising:
a cover member having flexibility and configured to cover the force sensation creating device and the supporting portion together with the target portion.

8. (Presently amended) The force module according to any one of claims 1, 2 and 5 to 7, wherein
the force sensation creating device comprises a vibrator, a linking mechanism connected to the vibrator, a sliding mechanism configured to guide a moving direction of the linking mechanism and the vibrator, a rotator connected to the linking mechanism, and a controller configured to drive the rotator to rotate.

9. (Presently amended) The force module according to any one of claims 1, 2 and 5 to 8, wherein
the supporting portion is formed of urethane foam.

10. (Presently amended) The force module according to any one of claims 1, 2 and 5 to 8, wherein
the supporting portion is formed of a flexible band member and shaped into a ring having a hollow portion.

11. (Presently amended) The force module according to any one of claims 1, 2 and 5 to 10, wherein
the supporting portion is sandwiched by, or adhered with an adhesive to, the force sensation creating device and the target portion.

12. (Presently amended) The force module according to any one of claims 1, 2 and 5 to 11, wherein
the target portion is a steering wheel of a vehicle equipped with a car navigation system, and
the force module comprises a controller configured to drive the force sensation creating device in such a manner as to create a being-pulled sensation based on a route indicated by the car navigation system.

13. (Presently amended) The force module according to any one of claims 1, 2 and 5 to 12, wherein
the target portion is a steering wheel of a vehicle, and
the force module is provided in an area of the steering wheel where a thumb, an index finger, or a middle finger of the user is placed.

14. (Presently amended) A haptic device comprising:
a target portion; and
a force module including a force sensation creating device arranged in the target portion and configured to create a kinesthetic illusion through asymmetric vibration, and a supporting portion arranged between the target portion and the force sensation creating device and configured to support the force sensation creating device with respect to the target portion in such a manner as to allow for vibration,
wherein the force sensation creating device is configured to generate asymmetric vibration in such a manner as to provide a being-pulled sensation in a predetermined guiding direction,
the target portion is an operation member manually manipulated by a user,
the guiding direction of the force sensation creating device is established along a manipulation direction of the target portion,
the target portion is a steering wheel that is rotationally manipulated, and
the force sensation creating device is arranged on an upper side of the steering wheel with respect to a rotational shaft of the steering wheel.

15. The haptic device according to claim 14, wherein the target portion is provided in the operation member that is manually manipulated, and the force module is arranged such that a vibration direction thereof is established along the manipulation direction.
